# FASCICULE DE BREVET EUROPEEN

(11) **EP 1 317 300 B1**
(45) Date de publication et mention de la délivrance du brevet: **17.08.2005**
(21) Numéro de dépôt: 01949581.1
(22) Date de dépôt: 29.06.2001
(51) Int. Cl.: A61M 5/32

(54) **DISPOSITIF DE PROTECTION D'UNE AIGUILLE DE SERINGUE**
SCHUTZVORRICHTUNG EINER SPRITZENNADEL
DEVICE FOR PROTECTING A SYRINGE NEEDLE

(30) Priorité: 09.08.2000 FR 0010473
(43) Date de publication de la demande: 11.06.2003
(73) Titulaire: Sanofi-Aventis, 75013 Paris (FR)
(72) Inventeur: BRUNEL, Marc, F-31000 Toulouse (FR)
(74) Mandataire: Cabinet BARRE LAFORGUE & associés
(86) Numéro de dépôt international: PCT/FR2001/002083
(87) Numéro de publication internationale: WO 2002/011799

(56) Documents cités:
- AT-B- 400 303
- FR-A- 2 777 787
- FR-A- 2 799 375
- US-A- 4 986 818

## Description

L'invention vise un dispositif de protection d'une aiguille de seringue, du type comportant un capuchon protège-aiguille en un matériau souple et un embout de protection en un matériau rigide adapté pour coiffer le capuchon protège-aiguille et doté de moyens internes de préhension dudit capuchon protège-aiguille aptes à permettre d'entraîner ce dernier lors du retrait dudit embout de protection.

De tels dispositifs de protection sont d'un usage courant et ont pour principal objectif, outre une notion de sécurité, de conduire à faciliter le retrait du capuchon protège-aiguille qui se trouve emprisonné dans l'embout de protection lors du retrait de ce dernier.

En vue d'emprisonner le capuchon protègé-aiguille, de façon usuelle, et comme décrit dans US-A-4 986 818, les embouts de protection sont dotés de moyens de préhension adaptés pour venir se positionner à l'arrière de la collerette bordant classiquement l'extrémité ouverte desdits capuchons protège-aiguille, de façon à venir buter contre ladite collerette et provoquer l'entraînement de ces capuchons lors du retrait des embouts de protection.

Bien que dans la pratique de tels embouts de protection permettent de remplir les objectifs visés, ils présentent toutefois un inconvénient majeur du fait qu'ils doivent être emboîtés en force sur le capuchon de façon que les moyens de préhension franchissent la collerette et, tendent, lors de cet emboîtement, à repousser ledit capuchon.

Or, le fait de repousser ainsi le capuchon peut avoir deux conséquences fâcheuses. En effet, et en premier lieu, il peut conduire à détériorer l'extrémité de l'aiguille piquée classiquement dans le capuchon. De plus, il peut entraîner une rupture de l'étanchéité entre le capuchon protège-aiguille et le nez de seringue au niveau du col d'étanchéité de cette dernière.

La présente invention vise à pallier ces inconvénients et a pour principal objectif de fournir un dispositif d'injection doté d'un embout de sécurité de protection du capuchon protège-aiguille permettant le retrait aisé de ce dernier, et non susceptible de détériorer l'aiguille et d'affecter l'étanchéité.

A cet effet, l'invention vise un dispositif de protection dont les moyens internes de préhension ménagés dans l'embout de protection comprennent au moins une griffe longitudinale solidarisée à la paroi latérale de l'embout de protection par le biais d'une charnière intermédiaire transversale souple formant une articulation délimitant longitudinalement :
- un tronçon longitudinal aval présentant une extrémité libre en forme de pointe apte à se ficher dans le capuchon protège-aiguille, ménagée de façon à venir au contact du capuchon protège-aiguille lors de l'introduction de ce dernier dans l'embout de protection et à entraîner le basculement de la griffe, ledit tronçon longitudinal aval comportant une face frontale, dite arrière, de butée contre la paroi latérale dudit embout de protection adaptée pour limiter ledit basculement,
- un tronçon longitudinal amont doté d'une face frontale, dite arrière, de butée contre la paroi latérale de l'embout de protection, adaptée pour limiter le basculement inverse de chaque griffe lors du retrait dudit embout de protection.

(Il est à noter que dans tout le texte de brevet, les termes amont, aval, postérieur, antérieur sont utilisés en prenant comme référence l'aiguille d'injection, les termes "aval" et "antérieur" désignant les portions des éléments les plus proches de cette aiguille d'injection, et les termes "amont" et "postérieur" les portions d'éléments les plus éloignées de cette dernière).

Selon l'invention, l'embout de protection est donc constitué d'une pièce unique intégrant au moins une griffe adaptée pour :
- pivoter d'un faible angle déterminé par la face de butée de son tronçon aval, lors de l'introduction du capuchon protège-aiguille, de façon que la pointe de chaque griffe vienne former une empreinte et se planter dans ledit capuchon protège-aiguille.
- pivoter en sens inverse, lors du retrait de l'embout de protection, d'un faible angle déterminé par la face de butée de son tronçon amont, de façon à augmenter la pénétration de la pointe de chaque griffe et à totalement immobiliser le capuchon protège-aiguille relativement audit embout de protection.

Le principe à la base de l'invention a donc été de concevoir un embout de protection dont chaque griffe peut pivoter par rapport à une charnière intermédiaire transversale entre deux positions extrêmes de butée déterminées par les faces de butée amont et aval de ladite griffe, qui permettent :
- une introduction du capuchon protège-aiguille à l'intérieur de l'embout de protection ne nécessitant pas d'effectuer d'effort notoire susceptible de repousser ledit capuchon, et au cours de laquelle la pointe de chaque griffe vient en contact intime avec la paroi de ce capuchon et se planter dans cette dernière,
- un retrait de l'embout de protection au cours duquel la pénétration de la pointe de chaque griffe se trouve augmentée, garantissant l'entraînement du capuchon protège-aiguille.

Il est, en outre, à noter que selon ce principe, la présence des faces de butée amont et aval est primordiale, car elles conduisent à limiter l'angle de pivotement de chaque griffe et garantissent le parfait accrochage et le maintien de chaque pointe de griffe dans le capuchon protège-aiguille, et ce malgré le fait que ce dernier soit réalisé en matériau souple.

Selon un mode de réalisation avantageux, chaque griffe comporte une face frontale, dite avant, de contact avec le capuchon protège-aiguille, présentant la forme d'une dièdre délimitant une arête longitudinale frontale adaptée pour venir en contact intime avec ledit capuchon protège-aiguille dans la position de butée du tronçon longitudinal aval de ladite griffe.

Cette disposition conduit, en effet, à augmenter la force de serrage de chaque griffe sur le capuchon protège-aiguille, du fait que la zone de contact entre ces derniers consiste en une arête venant s'imprimer dans la paroi dudit capuchon protège-aiguille.

De plus, de façon avantageuse, le tronçon aval de chaque griffe comporte une face frontale, dite arrière, présentant la forme d'un dièdre délimitant une arête longitudinale formant la pointe de ladite griffe avec l'arête longitudinale de la face frontale avant de cette dernière.

Par ailleurs, de façon usuelle, le capuchon protège-aiguille présente une forme tronconique. Dans ce cas et de façon avantageuse, l'arête longitudinale avant de chaque griffe est agencée de façon à être parallèle à la paroi périphérique dudit capuchon protège-aiguille, dans la position de butée du tronçon aval de ladite griffe.

Selon un autre mode de réalisation avantageux, le capuchon protège-aiguille comporte une pluralité de cannelures longitudinales aptes à loger chacune l'arête longitudinale avant d'une griffe.

Le mouvement naturel de retrait de l'embout de protection consistant à soumettre ce dernier à un mouvement de rotation assorti à un effort de traction, de telles cannelures dans lesquelles viennent se loger les arêtes frontales des griffes conduisent à fournir un système d'engrenage assurant le blocage en rotation du capuchon protège-aiguille à l'intérieur dudit embout de protection,

De plus, de façon avantageuse, chaque cannelure s'étend sur une longueur partielle du capuchon protège-aiguille de façon à ménager un épaulement de butée axiale de la pointe de chaque griffe. De tels épaulements dans lesquels viennent se ficher les pointes des griffes constituent une assurance supplémentaire garantissant le blocage en translation du capuchon protège-aiguille à l'intérieur de l'embout de protection.

De plus, en vue d'augmenter la hauteur de cet épaulement, et de façon avantageuse, le capuchon protège-aiguille comporte un renflement annulaire externe formant épaulement avec l'extrémité des cannelures.

Par ailleurs, à titre d'exemple de modes de réalisation avantageux :
- l'embout de protection comporte six griffes internes réparties autour de l'axe dudit embout de protection,
- le capuchon protège-aiguille comporte douze cannelures réparties autour de l'axe dudit capuchon,
- la charnière de chaque griffe est adaptée pour que la pointe de ladite griffe subisse un débattement transversal de l'ordre de 2mm, lors du pivotement de cette griffe entre ses deux positions de butée extrême.

Un tel dispositif de protection peut par ailleurs équiper une seringue logée dans un corps de seringue doté de moyens de blocage en rotation et en translation de ladite seringue. Dans ce cas, et de façon avantageuse, le corps de seringue comporte un tronçon sécable délimité par une zone frangible, constituant l'embout de protection.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins :
- la figure 1 est une vue en perspective d'un dispositif d'injection à usage unique incorporant un dispositif de protection conforme à l'invention,
- la figure 2 est une vue longitudinale partiellement en coupe par un plan longitudinal axial de l'élément de ce dispositif d'injection incorporant l'embout de protection conforme à l'invention,
- la figure 3 est une coupe longitudinale, à échelle agrandie, par un plan axial A du capuchon protège-aiguille de ce dispositif,
- la figure 4 est une coupe transversale par un plan B de ce capuchon protège-aiguille,
- la figure 5 est une vue en perspective, à échelle agrandie d'une des griffes de l'embout de protection,
- la figure 6 est une coupe transversale par un plan C de cet embout de protection,
- la figure 7 est une coupe longitudinale axiale du dispositif de protection selon l'invention monté sur une seringue pré-remplie,
- et la figure 8 est une coupe longitudinale axiale de ce dispositif d'injection lors du retrait de l'embout de protection.

Le dispositif de protection conforme à l'invention est représenté aux figures comme faisant partie intégrante d'un dispositif d'injection à usage unique comportant un corps de seringue 1 logeant une seringue pré-remplie 2 et doté de moyens de blocage en rotation et en translation de cette dernière.

Ce dispositif d'injection est du type décrit dans les demandes de brevets FR 2 799 375 et FR 2 799 376 auxquelles on se référera pour plus de détails.

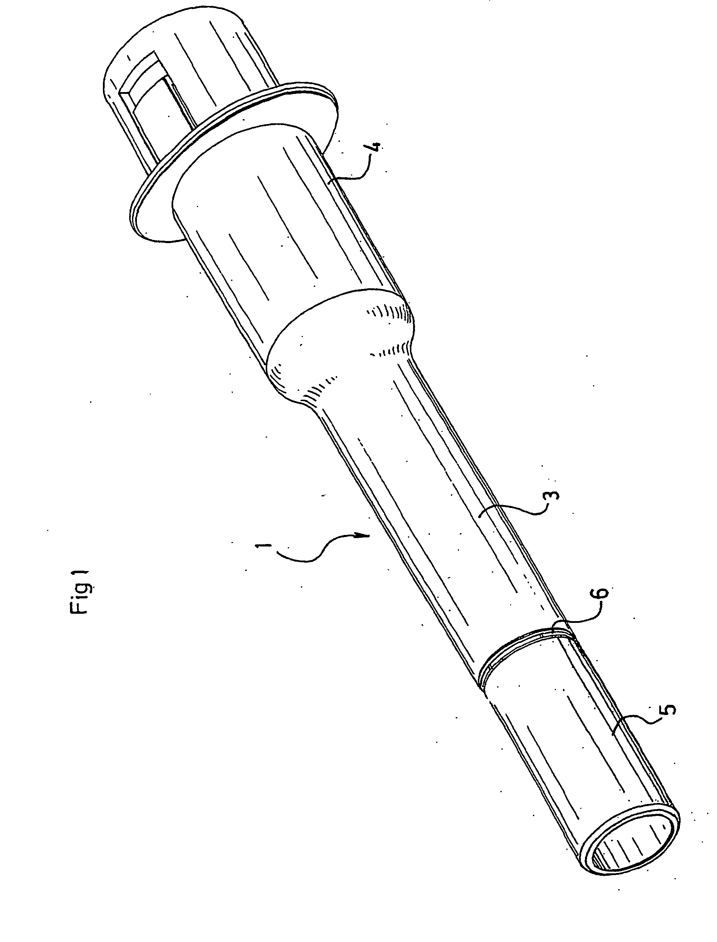

Tel que décrit dans ces demandes de brevet, ce dispositif d'injection comprend un corps de seringue constitué d'un étui protecteur 1, représenté à la figure 1, composé de deux corps tubulaires antérieur 3 et postérieur 4 adaptés pour venir s'emmancher dans le prolongement l'un de l'autre, et pour être pré-assemblés avant qu'une seringue classique 2 initialement pré-remplie soit mise en place dans cet étui protecteur 1.

Tel qu'également décrit dans ces demandes de brevet, ce dispositif d'injection incorpore des organes, dont des moyens élastiques, aptes à engendrer le retrait automatique de la seringue 2 à l'intérieur de l'étui protecteur 1 en fin d'injection.

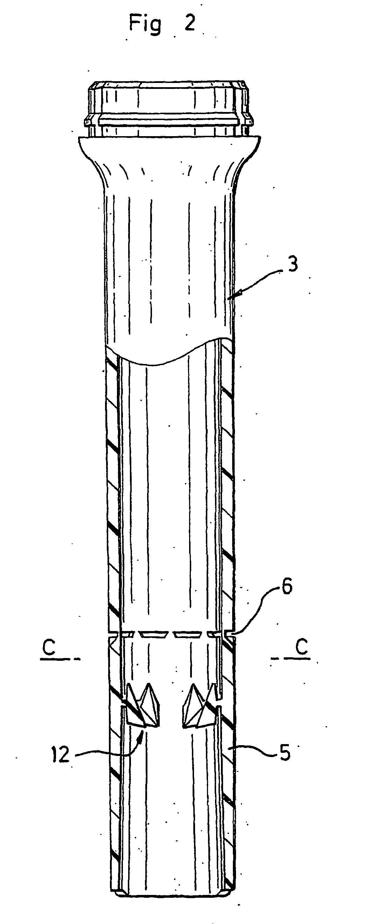

Tel que représenté notamment aux figures 1 et 2, et tel qu'également décrit dans les demandes de brevet précitées, le corps tubulaire antérieur 3 de l'étui protecteur 1 comporte un tronçon antérieur 5 sécable délimité par une zone annulaire 6 frangible, et formant l'embout de protection selon l'invention décrit en détail plus loin.

Tel que précité, la seringue pré-remplie 2 de ce dispositif d'injection est une seringue de type traditionnel, telle que par exemple réalisée en verre, comportant de façon classique un nez antérieur 2a sur lequel est montée une aiguille d'injection 7 protégée par un capuchon protège-aiguille 8.

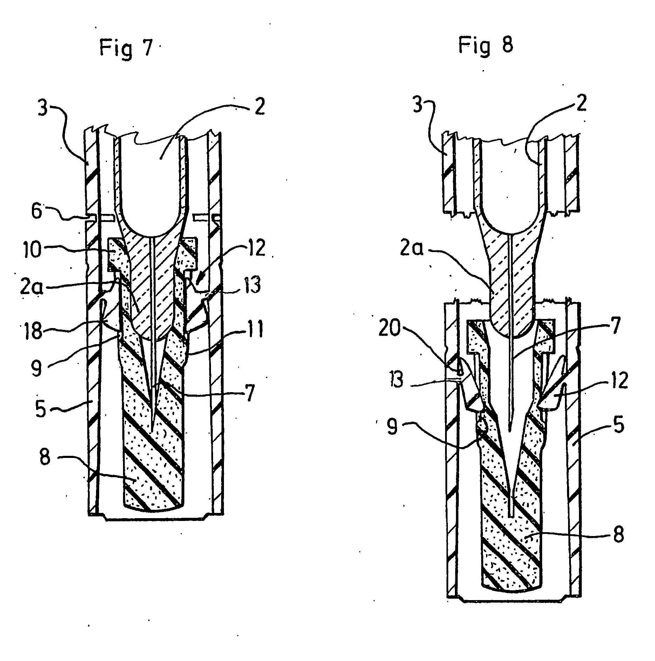

De plus, tel que représenté à la figure 7, et tel que décrit dans les demandes de brevet précitées, le corps de seringue 1 est adapté de façon que lors de la mise en place de la seringue 2 à l'intérieur dudit corps de seringue, le nez antérieur 2a de cette seringue 2 et le capuchon protège-aiguille 8 s'étendent dans l'embout de protection sécable 5.

Selon l'invention, et en premier lieu, le capuchon protège-aiguille 8 comporte une pluralité de cannelures longitudinales externes, telles que 9, ménagées à partir de la base antérieure de la collerette postérieure 10 dont est doté classiquement ledit capuchon, et s'étendant sensiblement sur le tiers de la longueur de ce capuchon protège-aiguille 8 à partir de ladite collerette.

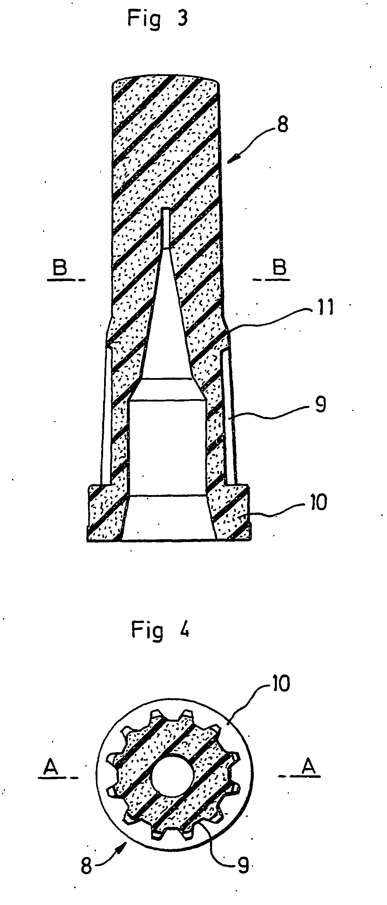

Tel que représenté à la figure 4, ces cannelures externes 9 sont en l'exemple au nombre de douze et sont uniformément réparties autour de l'axe longitudinal du capuchon protège-aiguille 8.

De plus, ce capuchon protège-aiguille 8 présente un renflement externe annulaire 11 formant un épaulement avec l'extrémité antérieure des cannelures 9.

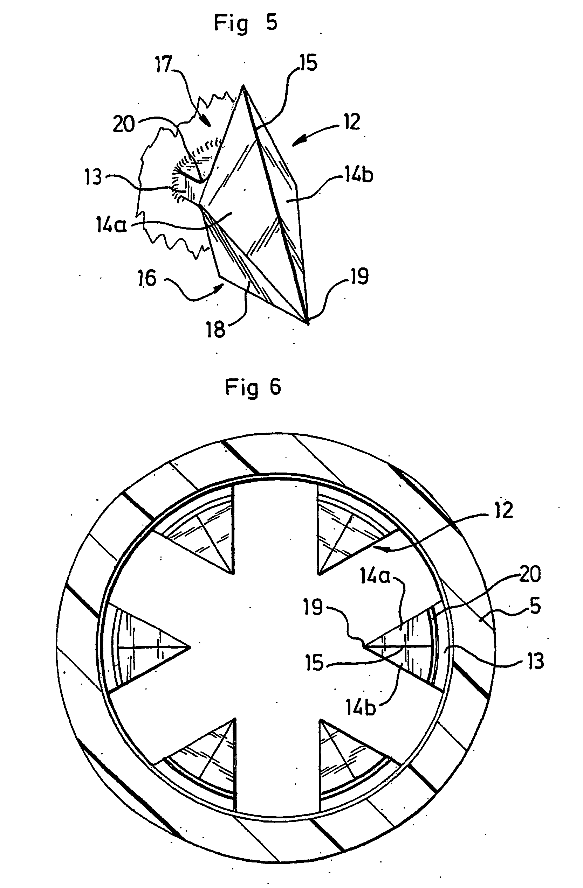

L'embout de protection 5 de ce protège-aiguille 8 composé du tronçon antérieur sécable du corps tubulaire 3, et destiné à faciliter le retrait du capuchon protège-aiguille 8, comporte quant à lui six griffes internes telles que 12 venues de moulage avec ledit embout de protection et représentées notamment aux figures 2, 5 et 6.

Ces six griffes internes 12, uniformément réparties autour de l'axe longitudinal de l'embout de protection 5 sont en premier lieu reliées à la face interne de la paroi latérale dudit embout de protection, par une charnière transversale intermédiaire 13 constituée d'un pied transversal souple autorisant un débattement angulaire desdites griffes.

Chacune de ces griffes 12 comporte, en premier lieu, une face frontale avant présentant la forme d'un dièdre dont chacune des faces 14a, 14b présente une forme triangulaire dont les bases forment l'arête longitudinale 15 dudit dièdre.

Chacune de ces griffes 12 comporte, en outre, un tronçon aval 16 et un tronçon amont 17 délimités par la charnière intermédiaire 13.

La face frontale arrière 18 du tronçon aval 16 présente la forme d'un dièdre adapté pour conférer audit tronçon aval, avec la face frontale avant 14a, 14b, une forme générale pyramidale dont le sommet forme une pointe d'extrémité 19 apte à se ficher dans le capuchon protège-aiguille 8.

De plus, cette face frontale arrière 18 constitue une face de butée contre la paroi latérale de l'embout de protection 5, apte à limiter le pivotement du tronçon aval 16 de la griffe 12.

La face frontale arrière 20 du tronçon amont 17 est quant à elle une face convexe de courbure conjuguée de celle de la paroi latérale de l'embout de protection 5, adaptée pour former une face de butée apte à limiter le pivotement dudit tronçon amont.

Tel que représenté à la figure 7, les griffes 12 sont positionnées de façon que la pointe 19 desdites griffes soit disposée sensiblement en amont de l'épaulement 11 du capuchon protège-aiguille, une fois la seringue 2 intégrée dans le corps de seringue 1.

De plus, les griffes 12 sont conformées de façon que le diamètre initial du cercle circonscrit par les pointes 19 desdites griffes soit inférieur au diamètre du capuchon protège-aiguille 8 de façon que ce dernier provoque le pivotement de ces griffes 12 lors de son introduction dans l'embout de protection 5.

Enfin, tel que représenté à la figure 7, la face de butée 18 du tronçon aval 16 et l'arête frontale 15 de chaque griffe 12, sont conformées de façon que ladite arête frontale s'étende parallèlement à la paroi périphérique du capuchon protège-aiguille 8, une fois la seringue 2 introduite dans le corps de seringue.

Selon le concept de l'invention, lors de l'introduction de la seringue 2 dans le corps de seringue 1, le capuchon protège-aiguille 8 vient au contact de la pointe 19 des griffes 12 et provoque le pivotement desdites griffes jusqu'à amener la face de butée 18 du tronçon aval 16 de ces dernières en butée contre la paroi latérale de l'embout de protection 5.

Lors de ce pivotement, les pointes 19 des griffes 12 viennent se ficher dans le capuchon protège-aiguille 8, sensiblement en amont de l'épaulement 11 de ce dernier. De plus, les arêtes longitudinales 15 de ces griffes 12 viennent se loger dans les cannelures 9 de ce capuchon protège-aiguille 8.

Par la suite, lors du retrait de l'embout de protection 5, en vue d'une injection, chaque griffe 12 est amené à pivoter en sens inverse de sorte que la pénétration de sa pointe 19 augmente et conduit à garantir l'entraînement du capuchon protège-aiguille 8.

De plus, lors de ce retrait, le blocage en rotation relatif du capuchon protège-aiguille 8 et de l'embout de protection 5 est assuré par l'engrenage formé par les cannelures 9 dans lesquelles sont logées les arêtes frontales 15 des griffes 12. L'épaulement 11 de l'embout de protection 5 garantit quant à lui le blocage relatif en translation de l'embout de protection 5 et du capuchon protège-aiguille 8.

Enfin, la face de butée 20 du tronçon amont 17 de chaque griffe 12, en limitant le pivotement de cette griffe 12, évite un éventuel retournement de ladite griffe, et garantit donc le blocage de cette dernière dans le capuchon protège-aiguille 8.

A titre d'exemple, le diamètre initial circonscrit par les pointes 19 des griffes 12 est de 4mm. Ce diamètre devient égal à 5mm dans la position de butée du tronçon aval 16 des griffes 12, puis égal à 3 mm dans la position de butée du tronçon amont 17 desdites griffes.

## Revendications

1. Dispositif de protection d'une aiguille (7) de seringue (2), comprenant un capuchon protège-aiguille (8) en un matériau souple et un embout de protection (5) en un matériau rigide adapté pour coiffer le capuchon protège-aiguille (8) et doté de moyens internes (12) de préhension dudit capuchon protège-aiguille aptes à permettre d'entraîner ce dernier lors du retrait dudit embout de protection, ledit dispositif de protection étant **caractérisé en ce que** les moyens internes de préhension ménagés dans l'embout de protection (5) comprennent au moins une griffe longitudinale (12) solidarisée à la paroi latérale de l'embout de protection (5) par le biais d'une charnière intermédiaire transversale souple (13) formant une articulation délimitant longitudinalement :
- un tronçon longitudinal aval (16) présentant une extrémité libre en forme de pointe (19) apte à se ficher dans le capuchon protège-aiguille (8), ménagée de façon à venir au contact dudit capuchon protège-aiguille lors de l'introduction de ce dernier dans l'embout de protection (5) et à entraîner le basculement de la griffe (12), ledit tronçon longitudinal aval comportant une face frontale (18), dite arrière, de butée contre la paroi latérale dudit embout de protection adaptée pour limiter ledit basculement,
- un tronçon longitudinal amont (17) doté d'une face frontale (20), dite arrière, de butée contre la paroi latérale de l'embout de protection (5), adaptée pour limiter le basculement inverse de chaque griffe (12) lors du retrait dudit embout de protection.

2. Dispositif de protection selon la revendication 1, **caractérisé en ce que** chaque griffe (12) comporte une face frontale (14a, 14b), dite avant, de contact avec le capuchon protège-aiguille (8), présentant la forme d'un dièdre délimitant une arête longitudinale (15) frontale adaptée pour venir en contact intime avec ledit capuchon protège-aiguille dans la position de butée du tronçon longitudinal aval (16) de ladite griffe.

3. Dispositif de protection selon la revendication 2, **caractérisé en ce que** le tronçon aval (16) de chaque griffe (12) comporte une face frontale (18), dite arrière présentant la forme d'un dièdre délimitant une arête longitudinale formant la pointe (19) de ladite griffe avec l'arête longitudinale (15) de la face frontale (14a, 14b) avant de cette dernière.

4. Dispositif de protection selon l'une des revendications 2 ou 3 dans lequel le capuchon protège-aiguille (8) présente une forme tronconique, **caractérisé en ce que** l'arête longitudinale avant (15) de chaque griffe (12) est agencée de façon à être parallèle à la paroi périphérique dudit capuchon protège-aiguille, dans la position de butée du tronçon aval (16) de ladite griffe.

5. Dispositif de protection selon l'une des revendications 2 à 4, **caractérisé en ce que** le capuchon protège-aiguille (8) comporte une pluralité de cannelures longitudinales aptes à loger chacune l'arête longitudinale avant (15) d'une griffe (12).

6. Dispositif de protection selon la revendication 5, **caractérisé en ce que** chaque cannelure (9) s'étend sur une longueur partielle du capuchon protège-aiguille (8) de façon à ménager un épaulement de butée axiale de la pointe (19) de chaque griffe (12).

7. Dispositif de protection selon la revendication 6, **caractérisé en ce que** le capuchon protège-aiguille (8) comporte un renflement annulaire externe (11) formant épaulement avec l'extrémité des cannelures (9).

8. Dispositif de protection selon l'une des revendications précédentes, **caractérisé en ce que** l'embout de protection (5) comporte six griffes internes (12) réparties autour de l'axe dudit embout de protection.

9. Dispositif de protection selon les revendications 5 et 8 prises ensemble, **caractérisé en ce que** le capuchon protège-aiguille (8) comporte douze cannelures (9) réparties autour de l'axe dudit capuchon.

10. Dispositif de protection selon l'une des revendications précédentes, **caractérisé en ce que** la charnière (13) de chaque griffe (12) est adaptée pour que la pointe (19) de ladite griffe subisse un débattement transversal de l'ordre de 2mm, lors du pivotement de cette griffe entre ses deux positions de butée extrême.

11. Combinaison d'un dispositif de protection selon l'une des revendications précédentes avec une seringue (2) logée dans un corps de seringue (1) doté de moyens de blocage en rotation et en translation de ladite seringue, **caractérisé en ce que** le corps de seringue (1) comporte un tronçon sécable (5) délimité par une zone frangible (6), constituant l'embout de protection.

## Claims

1. Device for protecting a syringe (2) needle (7), comprising a needle-protecting cap (8) made of a flexible material and a protective end-piece (5) made of a rigid material adapted to cover the needle-protecting cap (8) and equipped with internal means (12) for gripping the said needle-protecting cap which are capable of entraining the latter upon the removal of the said protective end-piece, the said protective device being **characterized in that** the internal gripping means provided in the protective end-piece (5) comprise at least one longitudinal claw (12) secured to the side wall of the protective end-piece (5) via a flexible transverse intermediate hinge (13) forming an articulation longitudinally delimiting:
- a downstream longitudinal section (16) having a free end in the form of a point (19) capable of sticking into the needle-protecting cap (8), and arranged so as to come into contact with the said needle-protecting cap upon the introduction of the latter into the protective end-piece (5) and to bring about the tilting of the claw (12), the said downstream longitudinal section having a rear end-face (18) for abutting against the side wall of the said protective end-piece, adapted to limit the said tilting,
- an upstream longitudinal section (17) equipped with a rear end-face (20) for abutting against the side wall of the protective end-piece (5), adapted to limit the opposite tilting of each claw (12) upon the removal of the said protective end-piece.

2. Protective device according to claim 1, **characterized in that** each claw (12) has a front end-face (14a, 14b) for contact with the needle-protecting cap (8), having the shape of a dihedron delimiting a frontal longitudinal edge (15) adapted to come into intimate contact with the said needle-protecting cap in the abutment position of the downstream longitudinal section (16) of the said claw.

3. Protective device according to claim 2, **characterized in that** the downstream section (16) of each claw (12) has a rear end-face (18) having the shape of a dihedron delimiting a longitudinal edge forming the point (19) of the said claw with the longitudinal edge (15) of the front end-face (14a, 14b) of the latter.

4. Protective device according to one of claims 2 and 3, in which the needle-protecting cap (8) has a frustoconical shape, wherein the front longitudinal edge (15) of each claw (12) is arranged so as to be parallel to the peripheral wall of the said needle-protecting cap, in the abutment position of the downstream section (16) of the said claw.

5. Protective device according to one of claims 2 to 4, **characterized in that** the needle-protecting cap (8) has a plurality of longitudinal grooves each capable of accommodating the front longitudinal edge (15) of a claw (12).

6. Protective device according to claim 5, **characterized in that** each groove (9) extends over a partial length of the needle-protecting cap (8) so as to provide a shoulder for axial abutment of the point (19) of each claw (12).

7. Protective device according to claim 6, **characterized in that** the needle-protecting cap (8) has an external annular bulge (11) forming a shoulder with the end of the grooves (9).

8. Protective device according to one of the preceding claims, **characterized in that** the protective end-piece (5) has six internal claws (12) distributed around the axis of the said protective end-piece.

9. Protective device according to claims 5 and 8 taken together, **characterized in that** the needle-protecting cap (8) has twelve grooves (9) distributed around the axis of the said cap.

10. Protective device according to one of the preceding claims, **characterized in that** the hinge (13) of each claw (12) is adapted so that the point (19) of the said claw undergoes a transverse displacement of the order of 2 mm upon the pivoting of this claw between its two end abutment positions.

11. Combination of a protective device according to one of the preceding claims, with a syringe (2) accommodated in a syringe body (1) equipped with means for rotational and translational locking of the said syringe, **characterized in that** the syringe body (1) has a separable section (5) delimited by a frangible zone (6), constituting the protective end-piece.

## Patentansprüche

1. Vorrichtung zum Schützen der Nadel (7) einer Spritze (2), die eine Nadelschutzkappe (8) aus einem elastischen Material und ein Schutzendstück (5) umfasst, das aus einem starren Material besteht und so gestaltet ist, dass es die Nadelschutzkappe (8) umgibt, und das mit inneren Greifmitteln (12) der genannten Nadelschutzkappe ausgestattet ist, so dass diese beim Zurückziehen des genannten Schutzendstücks mitgenommen werden kann, wobei die genannte Schutzvorrichtung **dadurch gekennzeichnet ist, dass** die in dem Schutzendstück (5) vorgesehenen inneren Greifmittel wenigstens eine Längsklaue (12) umfassen, die an der Seitenwand des Schutzendstückes (5) mittels eines elastischen transversalen Zwischenscharniers (13) angebracht sind, das ein Gelenk bildet, das longitudinal Folgendes begrenzt:
- einen unteren Längsabschnitt (16), der ein freies Ende (19) in der Form einer Spitze aufweist, das in die Nadelschutzkappe (8) passt und so gestaltet ist, dass es beim Einführen der genannten Nadelschutzkappe in das Schutzendstück (5) mit der Nadelschutzkappe in Kontakt kommt und die Klaue (12) kippt, wobei der genannte untere Längsabschnitt eine Stirnfläche (18), hintere Stirnfläche genannt, zur Anlage an der Seitenwand des genannten Schutzendstücks aufweist, um das genannte Kippen zu begrenzen,
- einen oberen Längsabschnitt (17), der eine Stirnfläche (20), hintere Stirnfläche genannt, zur Anlage an der Seitenwand des Schutzendstückes (5) aufweist, um ein umgekehrtes Kippen jeder Klaue (12) beim Zurückziehen des genannten Schutzendstückes zu begrenzen.

2. Schutzvorrichtung nach Anspruch 1, **dadurch gekennzeichnet, dass** jede Klaue (12) eine Stirnfläche (14a, 14b), vordere Stirnfläche genannt, für einen Kontakt mit der Nadelschutzkappe (8) aufweist, die die Form eines Dieders haben, das eine vordere Längskante (15) begrenzt, die so gestaltet ist, dass sie in der Anlageposition des unteren Längsabschnittes (16) der genannten Klaue mit der genannten Nadelschutzkappe in innigen Kontakt kommt.

3. Schutzvorrichtung nach Anspruch 2, **dadurch gekennzeichnet, dass** der untere Abschnitt (16) jeder Klaue (12) eine Stirnfläche (18), hintere Stirnfläche genannt, aufweist, die die Form eines Dieders hat, das eine Längskante begrenzt, die die Spitze (19) der genannten Klaue mit der Längskante (15) der Stirnfläche (14a, 14b) vor derselben bildet.

4. Schutzvorrichtung nach Anspruch 2 oder 3, in der die Nadelschutzkappe (8) kegelstumpfförmig ist, **dadurch gekennzeichnet, dass** die vordere Längskante (15) jeder Klaue (12) so gestaltet ist, dass sie parallel zur Umfangswand der genannten Nadelschutzkappe in der Anlageposition des unteren Abschnitts (16) der genannten Klaue ist.

5. Schutzvorrichtung nach einem der Ansprüche 2 bis 4, **dadurch gekennzeichnet, dass** die Nadelschutzkappe (8) eine Mehrzahl von Längsrillen aufweist, die jeweils eine vordere Längskante (15) einer Klaue (12) aufnehmen können.

6. Schutzvorrichtung nach Anspruch 5, **dadurch gekennzeichnet, dass** jede Rille (9) auf einer Teillänge der Nadelschutzkappe (8) verläuft, so dass sie einen axialen Längsansatz der Spitze (19) jeder Klaue (12) bildet.

7. Schutzvorrichtung nach Anspruch 6, **dadurch gekennzeichnet, dass** die Nadelschutzkappe (8) einen äußeren ringförmigen Wulst (11) aufweist, der einen Ansatz mit dem Ende der Rillen (9) bildet.

8. Schutzvorrichtung nach einem der vorherigen Ansprüche, **dadurch gekennzeichnet, dass** das Schutzendstück (5) sechs innere Klauen (12) aufweist, die um die Achse des genannten Schutzendstücks verteilt sind.

9. Schutzvorrichtung nach den Ansprüchen 5 und 8 zusammen genommen, **dadurch gekennzeichnet, dass** die Nadelschutzkappe (8) zwölf Rillen (9) aufweist, die um die Achse der genannten Kappe verteilt sind.

10. Schutzvorrichtung nach einem der vorherigen Ansprüche, **dadurch gekennzeichnet, dass** das Scharnier (13) jeder Klaue (12) so gestaltet ist, dass die Spitze (19) der genannten Klaue beim Schwenken dieser Klaue zwischen ihren beiden Endanlagepositionen einen transversalen Spielraum von etwa 2 mm hat.

11. Kombination einer Schutzvorrichtung nach einem der vorherigen Ansprüche mit einer Spritze (2), die im Spritzenkörper (1) aufgenommen wird, der mit Rotations- und Translationssperrmitteln der genannten Spritze ausgestattet ist, **dadurch gekennzeichnet, dass** der Spritzenkörper (1) einen teilbaren Abschnitt (5) aufweist, der von einer zerbrechlichen Zone (6) begrenzt wird, die das Schutzendstück bildet.
